# EUROPÄISCHE PATENTSCHRIFT

(11) **EP 1 252 128 B1**
(45) Veröffentlichungstag und Bekanntmachung des Hinweises auf die Patenterteilung: **11.08.2004**
(21) Anmeldenummer: 01915134.9
(22) Anmeldetag: 12.01.2001
(51) Int. Cl.: C07C 51/09, C07C 51/48, C07C 53/02

(54) **VERFAHREN ZUR GEWINNUNG VON WASSERFREIER AMEISENSÄURE**
METHOD FOR PRODUCING ANHYDROUS FORMIC ACID
PROCEDE D'OBTENTION D'ACIDE FORMIQUE ANHYDRE

(30) Priorität: 24.01.2000 DE 10002791
(43) Veröffentlichungstag der Anmeldung: 30.10.2002
(73) Patentinhaber: BASF AKTIENGESELLSCHAFT, 67056 Ludwigshafen (DE)
(72) Erfinder: AUER, Heinz, 68809 Neulussheim (DE); BESSLING, Bernd, Grosse Ille, MI 48138 (US); HAMMER, Hans, 68219 Mannheim (DE); HASSE, Hans, 67661 Kaiserslautern (DE); SAUER, Friedrich, 67271 Obersülzen (DE); VICARI, Maximilian, 67117 Limburgerhof (DE); ADRIAN, Till, 67240 Bobenheim-Roxheim (DE)
(74) Vertreter: Isenbruck, Günter, Dr.
(86) Internationale Anmeldenummer: PCT/EP2001/000344
(87) Internationale Veröffentlichungsnummer: WO 2001/053244

(56) Entgegenhaltungen:
- EP-A- 0 017 866
- EP-A- 0 596 484
- DE-A- 3 428 319

## Beschreibung

Die Erfindung betrifft ein Verfahren und eine Vorrichtung zur Gewinnung von wasserfreier oder weitgehend wasserfreier Ameisensäure.

Aus "Ullmanns Encyklopädie der technischen Chemie", 4. Auflage, Band 7, Seite 365, ist bekannt, Ameisensäure durch Acidolyse von Formamid mit Schwefelsäure herzustellen. Dieses Verfahren hat jedoch den Nachteil, daß stöchiometrische Mengen Ammoniumsulfat als Zwangsanfall erhalten werden.

Ein weitere Möglichkeit zur Herstellung von Ameisensäure besteht in der Hydrolyse von Methylformiat, das aus Methanol und Kohlenmonoxid synthetisiert wird. Dabei liegen die folgenden Gleichungen zugrunde:

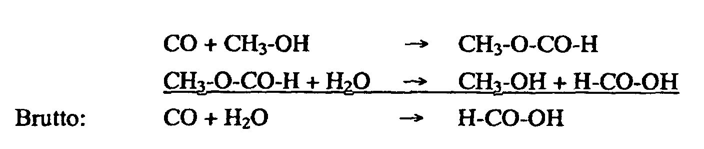

Die in "Ullmanns Encyklopädie der technischen Chemie", 4. Auflage, Band 7, Seite 366 beschriebene Hydrolyse von Methylformiat 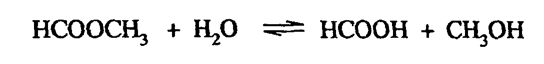 hat den Nachteil einer ungünstigen Lage des Hydrolysegleichgewichts. Eine Gleichgewichtsverschiebung durch destillative Entfernung der gewünschten Verfahrensprodukte ist nicht möglich, da Methylformiat (Sdp. 32°C) wesentlich niedriger siedet als Methanol (Sdp. 65°C) und Ameisensäure (Sdp. 101°C). Aus der anfallenden wäßrigen Ameisensäurelösung kann wasserfreie Ameisensäure wegen Azeotropbildung mit Wasser nicht ohne weiteres destillativ gewonnen werden. Die Schwierigkeit besteht also darin, aus dem Hydrolysegemisch des Methylformiats wasserfreie Ameisensäure zu gewinnen.

Ein in der EP-B-0 017 866 beschriebenes, die wichtigen Verfahrensschritte a) bis g) aufweisendes Verfahren ermöglicht ausgehend von Methylformiat die Herstellung wasserfreier Ameisensäure. Dabei erhält man wasserfreie Ameisensäure, falls man
a) Methylformiat der Hydrolyse unterwirft,
b) vom erhaltenen Hydrolysegemisch Methanol sowie überschüssiges Methylformiat abdestilliert,
c) das Ameisensäure und Wasser enthaltende Sumpfprodukt der Destillation (b) in einer Flüssig-flüssig-Extraktion mit einem hauptsächlich die Ameisensäure aufnehmenden Extraktionsmittel extrahiert,
d) die hierbei erhaltene Ameisensäure, Extraktionsmittel und eine Teilmenge des Wassers aufweisende Extraktphase einer Destillation unterwirft,
e) das bei dieser Destillation erhaltene Wasser und eine Teilmenge der Ameisensäure enthaltende Kopfprodukt in den unteren Teil der Destillationskolonne der Stufe (b) zurückführt,
f) das vorwiegend Extraktionsmittel und Ameisensäure enthaltende Sumpfprodukt der Destillationsstufe (d) destillativ in wasserfreie Ameisensäure und das Extraktionsmittel auftrennt und
g) das die Stufe (f) verlassende Extraktionsmittel in den Verfahrensgang zurückführt.

Es ist bei diesem Verfahren besonders zweckmäßig,
h) die Destillationsschritte (b) und (d) in einer einzigen Kolonne vorzunehmen,
i) das für die Hydrolyse benötigte Wasser dampfförmig in den unteren Teil der für die Durchführung von Schritt (b) vorgesehenen Kolonne einzubringen,
k) Methylformiat und Wasser bei der Hydrolyse (a) im Molverhältnis 1:2 bis 1:10 einzusetzen und/oder
l) als Extraktionsmittel ein Carbonsäureamid der allgemeinen Formel I 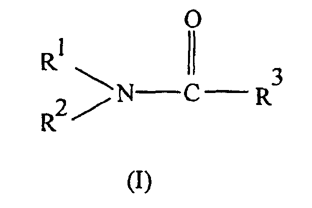 einzusetzen, in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5- oder 6-Ring ausbilden und daß nur einer der Reste eine Arylgruppe ist und in der R³ für Wasserstoff oder eine C₁-C₄-Alkylgruppe steht.

Im folgenden werden die Verfahrensschritte (a) bis (i) näher erläutert.

### Verfahrensschritt (a)

Die Hydrolyse wird üblicherweise in einem Temperaturbereich von 80 bis 150°C durchgerührt.

### Verfahrensschritt (b)

Die Destillation des Hydrolysegemisches kann prinzipiell bei beliebigem Druck, bevorzugt 0,5 bis 2 bar, vorgenommen werden. Im allgemeinen empfiehlt sich das Arbeiten unter Normaldruck. In diesem Fall beträgt die Temperatur im Kolonnensumpf etwa 110°C und am Kolonnenkopf etwa 30 bis 40°C. Das Hydrolysegemisch wird zweckmäßigerweise in einem Temperaturbereich von 80 bis 150°C zugegebenen, und das Methanol entnimmt man vorzugsweise flüssig bei Temperaturen von 55 bis 65°C. Eine zufriedenstellende Trennung des Gemisches in Methylformiat sowie Methanol einerseits und die wäßrige Ameisenäure andererseits ist bereits mit einer Destillationskolonne möglich, die 25 theoretischen Böden aufweist (bevorzugt ist eine theoretische Bodenzahl von 35 bis 45). Die Bauart der für den Verfahrensschritt (b) vorgesehenen Kolonne kann beliebig sein, besonders empfiehlt sich jedoch eine Siebboden- oder Füllkörper-Kolonne.

### Verfahrensschritt (c)

Die Flüssig-flüssig-Extraktion der Ameisensäure aus ihrer wäßrigen Lösung mittels eines Extraktionsmittels wird vorzugsweise bei Normaldruck und bei Temperaturen von 60 bis 120, insbesondere 70 bis 90°C im Gegenstrom vorgenommen. Je nach Art des Extraktionsmittels benötigt man in der Regel Extraktionseinrichtungen mit 1 bis 12 theoretischen Trennstufen. Geeignete Extraktionseinrichtungen dafür sind insbesondere Flüssig-flüssig-Extraktionskolonnen. In den meisten Fällen erzielt man mit 4 bis 6 theoretischen Trennstufen befriedigende Ergebnisse.

Die Wahl des Extraktionsmittels ist nicht beschränkt. Als Extraktionsmittel besonders geeignet sind Carbonsäureamide der vorstehend genannten allgemeinen Formel I. Derartige Extraktionsmittel sind vor allem N-Di-n-butylformamid sowie außerdem N-Din-butylacetamid, N-Methyl-N-2-heptylformamid, N-n-Butyl-N-2-ethylhexylformamid, N-n-Butyl-N-cyclohexylformamid und N-Ethylformanilid sowie Gemische dieser Verbindungen. Weitere geeignete Extraktionsmittel sind u.a. Diisopropylether, Methylisobutylketon, Ethylacetat, Tributylphosphat und Butandiolformiat.

### Verfahrensschritt (d)

Die Extraktphase wird in einer entsprechenden Destillationseinrichtung destillativ in eine Flüssigphase, die in der Regel vorwiegend Ameisensäure und Extraktionsmittel aufweist, sowie eine vorwiegend Wasser und geringe Mengen Ameisensäure aufweisende Dampfphase getrennt. Es handelt sich dabei um eine Extraktivdestillation. Die Sumpftemperatur beträgt vorzugsweise 140 bis 180°C. Ein ausreichender Trenneffekt wird in der Regel ab 5 theoretischen Böden erzielt.

### Verfahrensschritt (e)

Die Rückführung des Ameisensäure-Wassergemischs erfolgt in der Regel dampfförmig.

### Verfahrensschritte (f) und (g)

Die Destillationseinrichtung (meist als Kolonne ausgebildet) zur Durchführung der Stufe (f) wird zweckmäßigerweise unter vermindertem Druck - etwa 50 bis 300 mbar und entsprechend niedrigen Kopftemperaturen - etwa 30 bis 60°C, betrieben.

### Verfahrensschritt (h)

Diese Variante des Verfahrens betrifft die Schritte (b) und (d). Die Destillationseinrichtungen zur Durchführung der Stufen b) und d) werden in einer Gesamtdestillationseinrichtung angeordenet. Die Destillationseinrichtungen sind dabei in der Regel als Kolonnen ausgebildet.

### Verfahrensschritt (i)

In diesem Schritt wird für die Hydrolyse benötigtes Wasser in Form von Wasserdampf bereitgestellt.

Die Synthese von Methylformiat aus Kohlenmonoxid und Methanol wird in der Technik mit einem molaren Überschuß Methanol durchgeführt. Im Reaktoraustrag des entsprechenden Synthesereaktors sind deshalb neben Methylformiat noch erhebliche Mengen an überschüssigem Methanol enthalten. Der Reaktoraustrag enthält üblicherweise zwischen 20 und 40 Gew.-% Methanol - der verbleibende Teil besteht im wesentlichen aus Methylformiat. Die Wirtschaftlichkeit des in der EP-B-0 017 866 beschriebenen Verfahrens wird dadurch verbessert, daß man Methanol in dem Synthesereaktor entnommenen, Methylformiat und Methanol enthaltenden Gemisch abreichert und erst dann dem Hydrolysereaktor zur Durchführung der Stufe a) zuführt. Es ist wirtschaftlich am sinnvollsten, einen Methylformiatstrom in den Hydrolysereaktor einzuleiten, der etwa 95 % Methylformiat enthält - der Methanolgehalt ist demgemäß in diesem Strom deutlich niedriger als in dem Strom, der den Synthesereaktor verläßt. Die Abreicherung von Methanol geschieht in einer Destillationskolonne, die mit einem Verstärkungsteil ausgestattet ist und am Kopf einen Rücklauf aufweist. Die Verdampfungsenthalpie des Rücklaufs dieser Kolonne muß im Sumpfverdampfer in Form von Dampf eingetragen werden, d.h. es muß zusätzlich Energie in den Prozeß eingetragen werden. Neben diesem Nachteil der hohen Energiekosten wird die Wirtschaftlichkeit des Verfahrens auch durch die hohen Investitionskosten für die entsprechende Kolonne negativ beeinflußt. Die Kosten für die Bereitstellung von Methylformiat, das nur geringe Mengen Methanol (z.B. 5 Gew.%) enthält, schmälert somit den wirtschaftlichen Vorteil, der sich aus dem Einsatz von hochkonzentriertem Methylformiat ergibt.

EP-A 0 596 484 betrifft ein Verfahren zur Herstellung von Ameisensäure, wobei Methylformiat mit Wasser in Gegenwart eines stark sauren Katalysators (Sulfokationenaustauscher) hydrolysiert wird. Bei diesem Verfahren wird im Anschluss an die Hydrolyse von (methanolhaltigem) Methylformiat das Reaktionsprodukt in eine Rektifikationskolonne geleitet, um das Verfahrensprodukt (Ameisensäure) vom nicht umgesetzten Edukt sowie Methanol abzutrennen. Dabei wird das Methanol/Methylformiat-Gemisch in eine weitere Kolonne überführt, in der Methylformiat abgetrennt und in den Hydrolysereaktor rückgeführt wird.

DE-A 34 28 319 beschreibt ein Verfahren zur Gewinnung wasserfreier beziehungsweise weitgehend wasserfreier Ameisensäure. Ähnlich zu EP-A 0 596 484 wird im Anschluss an die Hydrolyse von Methylformiat Methanol sowie nicht umgesetztes Methylformiat in einer Destillationskolonne von der Ameisensäure abgetrennt. Das Destillat wird in einer weiteren Kolonne in Methanol sowie Methylformiat aufgetrennt, die an jeweils verschiedenen Stellen in den Prozess zur Herstellung von Ameisensäure rückgeführt werden.

Die Aufgabe der vorliegenden Erfindung besteht darin, ein Verfahren bereitzustellen, bei dem man wasserfreie oder weitgehend wasserfreie Ameisensäure gewinnt. Die Wirtschaftlichkeit des Verfahrens soll dadurch verbessert werden, daß der energetische, sowie apparative Aufwand für die Abreicherung von Methanol in dem für die Hydrolyse eingesetzten, Methylformiat und Methanol enthaltenden Gemisch reduziert wird.

Die Lösung dieser Aufgabe geht aus von dem Verfahren zur Gewinnung von wasserfreier oder weitgehend wasserfreier Ameisensäure, bei dem man
i) Methylformiat der Hydrolyse unterwirft,
ii) vom erhaltenen Hydrolysegemisch Methanol sowie überschüssiges Methylformiat abdestilliert,
iii) das Ameisensäure und Wasser enthaltende Sumpfprodukt der Destillation ii) in einer Flüssig-flüssig-Extraktion mit einem hauptsächlich die Ameisensäure aufnehmenden Extraktionsmittel extrahiert,
iv) die hierbei erhaltene, Ameisensäure, Extraktionsmittel und eine Teilmenge des Wassers aufweisende Extraktphase einer Destillation unterwirft,
v) das bei dieser Destillation erhaltene, Wasser und eine Teilmenge der Ameisensäure enthaltene Kopfprodukt in den unteren Teil der Destillationseinrichtung der Stufe ii) zurückführt,
vi) das vorwiegend Extraktionsmittel und Ameisensäure enthaltende Sumpfprodukt der Destillationsstufe iv) destillativ in wasserfreie oder weitgehend wasserfreie Ameisensäure und das Extraktionsmittel auftrennt und
vii) das die Stufe vi) verlassende Extraktionsmittel in den Verfahrensgang zurückführt.

Das erfindungsgemäße Verfahren ist dadurch gekennzeichnet, daß vor der Hydrolyse des Methylformiats (Stufe i)), Methanol enthaltendes Methylformiat in die zur Durchführung der Stufe ii) vorgesehene Destillationseinrichtung geführt wird, wobei sich die entsprechende Zufuhrstelle des Methanol enthaltenden Methylformiats in der Destillationseinrichtung oberhalb der Entnahmestelle für Methanol und unterhalb der Entnahmestelle für Methylformiat befindet und an der Entnahmestelle für Methylformiat an Methanol abgereichertes Methylformiat erhalten wird, das nachfolgend der Stufe i) zugeführt wird.

Unter weitgehend wasserfreier Ameisensäure soll Ameisensäure verstanden werden, die bis zu maximal 30 Gew.-%, bevorzugt bis zu maximal 15 Gew.-%, Wasser enthält. Als an Methanol abgereichertes Methylformiat soll Methylformiat verstanden werden, das weniger Methanol enthält, als das den Synthesereaktor verlassende Methylformiat.

Ein wesentlicher Vorteil ist es, daß auch Methylformiat mit einem höheren Anteil an Methanol (zum Beispiel 30 %) für das erfindungsgemäße Verfahren bereitgestellt werden kann. Dadurch verringern sich der energetische sowie der apparative Aufwand, und das Verfahren kann somit wirtschaftlicher betrieben werden. Ein wesentlicher Vorteil ist es also, daß zur Aufkonzentrierung des Methylformiats aus dem Synthesereaktor eine einfache als Abtriebskolonne ausgebildete Destillationskolonne (ohne Verstärkungsteil und Rücklauf) ausreicht, wenn der so gewonnene, teilweise aufkonzentrierte Methylformiat-Strom in die Destillationseinrichtung ii eingespeist wird, wo er ohne zusätzlichen Energieaufwand auf die z.B. gewünschten 95 % aufkonzentriert werden kann.

In einer bevorzugten Ausführungsform der Erfindung wird das Methanol aufweisende Methylformiat dadurch erhalten, daß man dem Synthesereaktor entnommenes, Methylformiat und Methanol enthaltendes Gemisch einer bevorzugt als Abtriebskolonne ausgebildeten Destillationskolonne zuführt, dort eine Teilmenge des Methanols abtrennt und am oberen Ende der Destillationskolonne das Methanol aufweisende Methylformiat entnimmt. In der Regel weist die bevorzugt als Abtriebskolonne ausgebildete Destillationskolonne keinen Verstärkungsteil auf. Bevorzugt wird auf einen Rücklauf im oberen Teil der Destillationskolonne verzichtet. Eine solche Destillationskolonne kann den Methanolgehalt im Methylformiat, das anschließend in die Destillationseinrichtung zur Durchführung der Stufe ii) geführt wird, in ausreichendem Maße abreichem. Besonders vorteilhaft an einer solchen Destillationskolonne sind die geringeren Baukosten, die insbesondere dadurch minimiert werden können, daß auf einen Verstärkungsteil und einen Rücklauf verzichtet wird. Durch die Reduzierung der Heizdampfmenge kann außerdem die Größe der Wärmeüberträger der Kolonne und der Kühlwasserbedarf des entsprechenden Kondensators verringert werden.

Vorteilhafterweise werden die Destillationsschritte ii) und iv) in einer einzigen Destillationseinrichtung durchgeführt. Diese erfüllt entsprechend die Funktionen der einzelnen Destillationskolonnen. Bevorzugt wird das für die Hydrolyse (Schritt i)) benötigte Wasser dampfförmig in den unteren Teil der Destillationseinrichtung geführt, die für die Durchführung von Schritt ii) vorgesehen ist.

Erfindungsgemäß wird auch eine Vorrichtung zur Durchführung des vorstehend beschriebenen Verfahrens bereitgestellt. Diese enthält,
α) einen Synthesereaktor,
β) einen Hydrolysereaktor,
χ) eine Destillationseinrichtung zur Durchführung der Stufe ii), aufweisend eine Zufuhrstelle für Methanol enthaltendes Methylformiat und Entnahmestellen für Methanol und Methylformiat,
δ) eine Destillationseinrichtung zur Durchführung der Stufe iv),
ε) eine Extraktionseinrichtung und
φ) eine Destillationseinrichtung zur Durchführung der Stufe vi).

Als Synthesereaktoren eignen sich alle Reaktoren, die für die Herstellung von Methylformiat ausgehend von Methanol und Kohlenmonoxid geeignet sind. Die Destillationseinrichtungen χ), δ) und φ) sind in der Regel als Kolonnen ausgebildet. Als Extraktionseinrichtung wird bevorzugt eine Flüssig-flüssig-Extraktionskolonne eingesetzt.

In einer bevorzugten Ausführungsform enthält die erfindungsgemäße Vorrichtung neben den Elementen α) bis φ) außerdem noch eine Destillationskolonne, die bevorzugt als Abtriebskolonne ausgebildet ist, d.h. im oberen Teil weder einen Verstärkungsteil besitzt, noch mit einem Rücklauf beaufschlagt wird. Die bevorzugt als Abtriebskolonne ausgebildete Destillationskolonne dient zur Abtrennung von überschüssigem Methanol aus dem Reaktionsgemisch des Synthesereaktors.

Bevorzugt ist die Destillationseinrichtung zur Durchführung der Stufe ii) und die Destillationseinrichtung zur Durchführung der Stufe iv) in einer einzigen Destillationseinrichtung angeordnet. Letztere ist meist als eine große Kolonne ausgebildet, die im oberen Teil die Destillationseinrichtung zur Durchführung der Stufe ii) und im unteren Teil die Destillationseinrichtung zur Durchführung der Stufe iv) aufweist.

Die Zeichnung zeigt
- in Fig. 1: ein Schema einer Anlage zur Herstellung von wasserfreier bzw. weitgehend wasserfreier Ameisensäure nach dem Stand der Technik,
- in Fig. 2: ein Schema zur Herstellung von wasserfreier bzw. weitgehend wasserfreier Ameisensäure nach dem Stand der Technik, wobei die Destillationseinrichtung zur Durchführung der Stufe ii) und die Destillationseinrichtung zur Durchführung der Stufe iv) in einer einzigen Destillationseinrichtung angeordnet sind,
- in Fig. 3: ein Schema einer Anlage zur Durchführung des erfindungsgemäßen Verfahrens zur Herstellung von wasserfreier, bzw. weitgehend wasserfreier Ameisensäure und
- in Fig. 4: ein Schema einer Anlage zur Durchführung des erfindungsgemäßen Verfahrens zur Herstellung von wasserfreier, bzw. weitgehend wasserfreier Ameisensäure, wobei die Destillationseinrichtung zur Durchführung der Stufe ii) und die Destillationseinrichtung zur Durchführung der Stufe iv) in einer einzigen Destillationseinrichtung angeordnet sind.

Die über bzw. neben den Pfeilen eingetragenen Bezugszeichen geben diejenigen Komponenten an, die im allgemeinen in den jeweiligen Strömen einen hohen Anteil bzw. den Hauptanteil aufweisen. Da die Anteile der Komponenten in den Strömen variieren können, sollten diese Bezugszeichen nur als Richtwert zur Orientierung dienen. Bezugszeichen 21 steht für Methylformiat, 22 für Wasser, 23 für Ameisensäure, 24 für Methanol, 25 für Extraktionsmittel, 26 für Abgas und 27 für Kohlenmonoxid. Den Verfahren nach dem Stand der Technik und dem erfindungsgemäßen Verfahren ist gemeinsam, daß Methylformiat in einem Synthesereaktor 6 hergestellt, die Hydrolyse des Methylformiats in einem Hydrolysereaktor 1 durchgeführt und die Durchführung der Stufe ii) in einer Destillationseinrichtung 2 vorgenommen wird, die Extraktion in einer Extraktionseinrichtung 3 erfolgt, die Durchführung der Stufe iv) in einer Destillationseinrichtung 4 erfolgt und Stufe vi) in einer Destillationseinrichtung 5 vorgenommen wird.

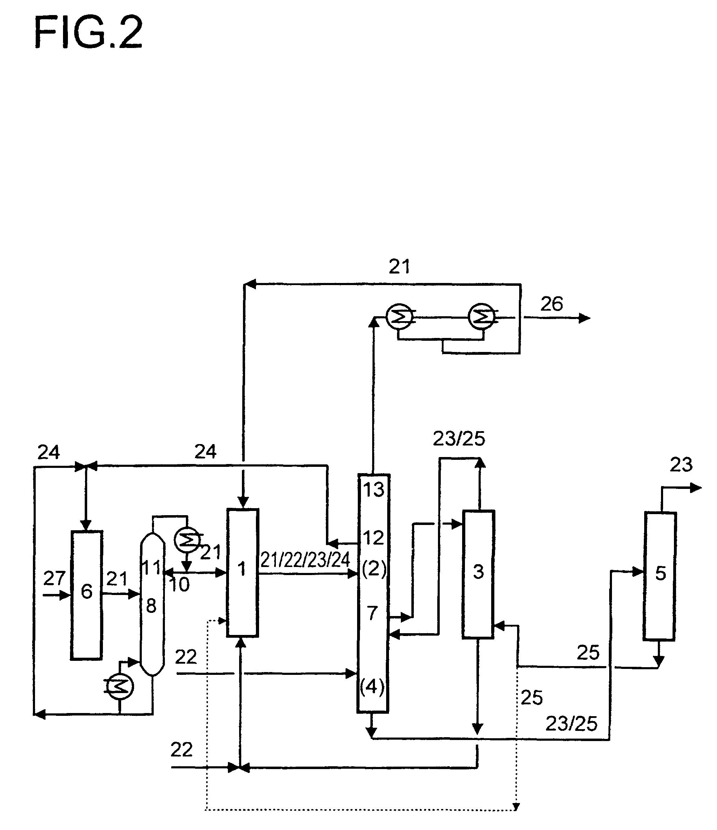

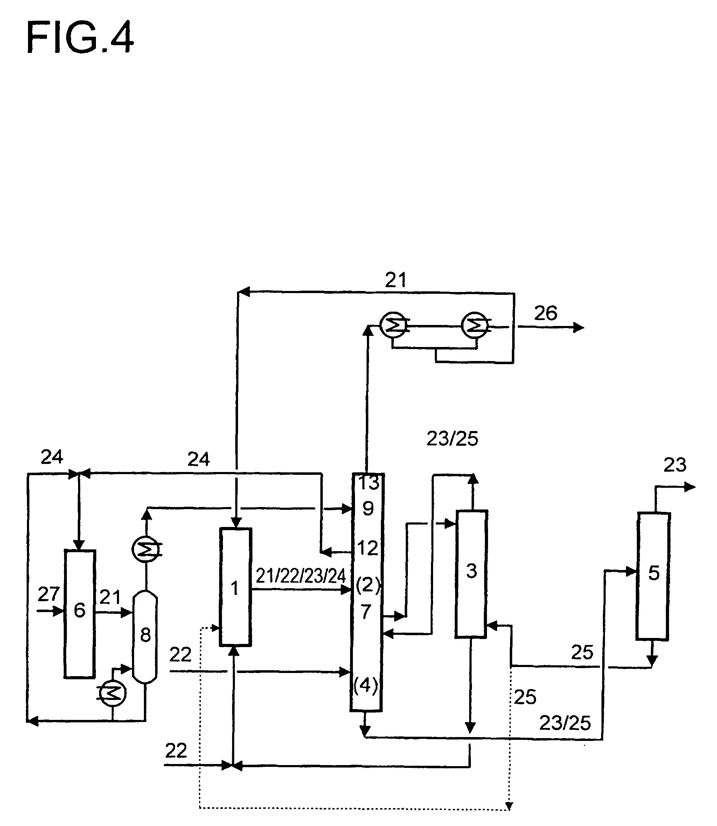

In Fig. 2 und Fig. 4 sind die Destillationseinrichtungen 2; 4 in einer einzigen Destillationseinrichtung 7 angeordnet. Die erfindungsgemäßen und die Verfahren nach dem Stand der Technik unterscheiden sich dadurch, daß der aus dem Synthesereaktor ausgeführte Methylformiat und Methanol enthaltende Strom in unterschiedlich ausgestaltete Destillationskolonnen 8 zugeführt wird. Die Destillationskolonnen nach dem Stand der Technik enthalten einen Verstärkungsteil 11 und einen Rücklauf 10 - bei den Anlagen für das erfindungsgemäße Verfahren fehlen diese Elemente. Das die Destillationskolonne 8 verlassende, eine Restmenge an Methanol aufweisende Methylformiat wird gemäß dem Verfahren nach dem Stand der Technik direkt in den Hydrolysereaktor 1 gerührt, Im Gegensatz dazu wird das die Destillationskolonne 8 verlassende, Methanol aufweisende Methylformiat bei den erfindungsgemäßen Verfahren der Destillationseinrichtung 2 zugeführt, wobei die entsprechende Zufuhrstelle 9 oberhalb der Entnahmestelle für Methanol 12 und unterhalb der Entnahmestelle für Methylformiat 13 angeordnet ist. Das an der Entnahmestelle 13 für Methylformiat erhaltene Methylformiat ist in der Destillationseinrichtung 2 an Methanol abgereichert worden. Dieses an Methanol abgereicherte Methylformiat wird dann dem Hydrolysereaktor 1 zugeführt.

Die Erfindung soll im folgenden anhand eines Ausführungsbeispiels näher erläutert werden.

### Vergleichsbeispiel

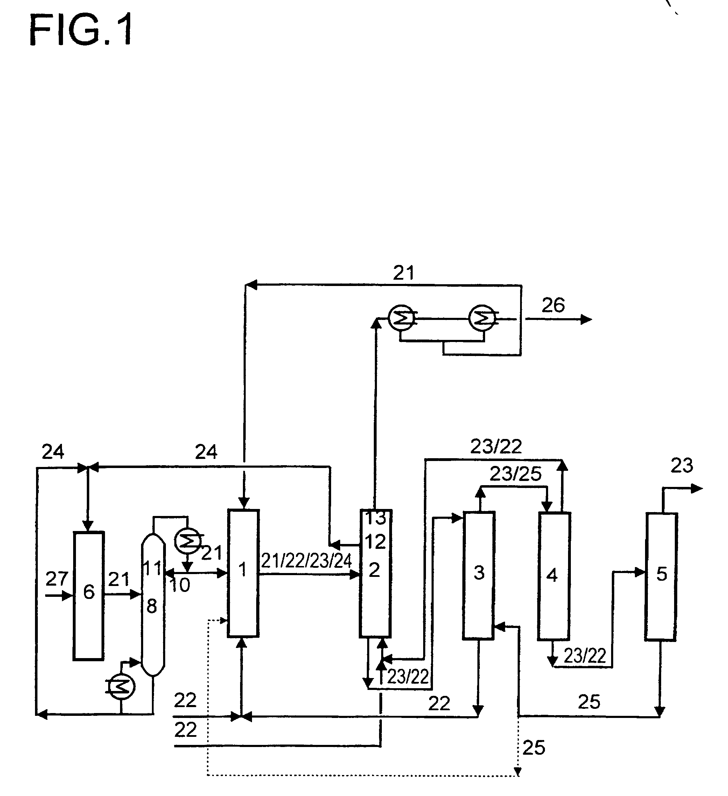

Das Vergleichsbeispiel entspricht einem Verfahren nach dem Stand der Technik, das in einer Anlage durchgeführt wird, die schematisch in Fig. 1 aufgezeigt ist. Es werden 5,3 kg wässrige Ameisensäure hergestellt. Das den Synthesereaktor 6 verlassende Methylformiat und Methanol enthaltende Gemisch wird in die Destillationskolonne 8 mit dem Verstärkerteil 11 geführt, wobei in dieser Kolonne Methylformiat bis 95 Gew.-% aufkonzentriert wird. Letzteres wird in den Hydrolysereaktor 1 geführt. Das Reaktionsprodukt des Synthesereaktors enthält neben dem Methylformiat 25 Gew.-% Methanol. Die den Verstärkungsteil 11 aufweisende Destillationskolonnne 8 ist als Glockenbodenkolonne mit insgesamt 30 Böden ausgebildet. Das aus dem Synthesereaktor 6 entnommene Gemisch wird auf Boden 15 aufgegeben. Die Glockenbodenkolonne enthält einen Sumpfverdampfer, der 0,9 kW Wärme einträgt und einen Kopfkondensator, der 0,95 kW Wärme abzieht. Die Destillationseinrichtung 2 ist als Glasglockenkolonne mit 80 Böden ausgebildet. Der Zulauf des aus dem Hydrolysereaktor 1 entnommenen Gemischs erfolgt auf Boden 15. In den Sumpf der Destillationseinrichtung 2 wurde zusätzlich der kondensierte, flüssige Kopfstrom der Destillationseinrichtung 4 eingetragen. Flüssiges Methanol für die Rezirkulierung in die Methylformiatsynthese wird an der Entnahmestelle 12 abgezogen - die Entnahmestelle 12 befindet sich auf dem Boden 50. Am Kopf der Destillationseinrichtung 2 wird an der Entnahmestelle 13 95 Gew.-%iges Methylformiat abgezogen. Dazu müssen in dem Sumpfverdampfer der entsprechenden Destillationseinrichtung 7 4,9 kW Wärme eingetragen werden. Am Kopfkondensator werden 4,5 kW Wärme abgezogen.

### Beispiel

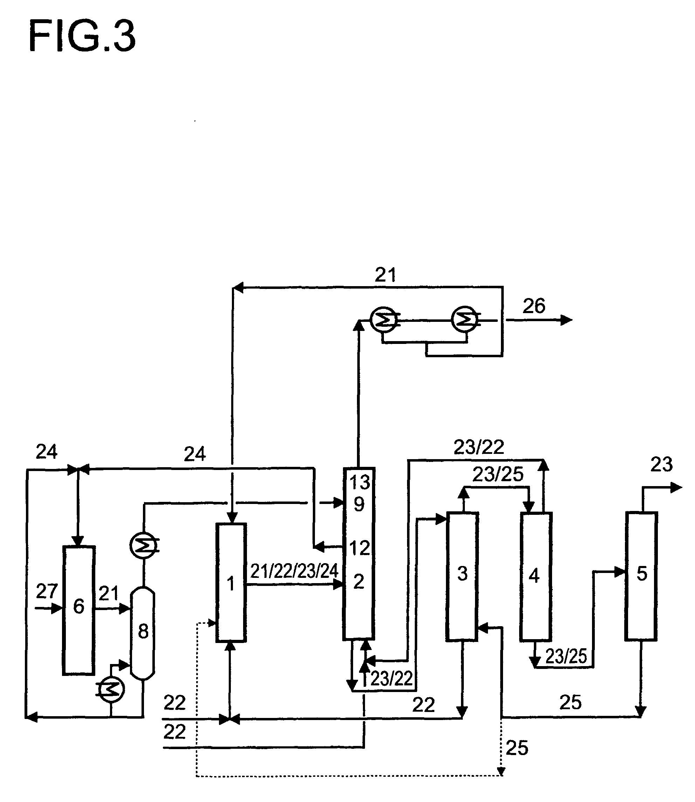

Der nachstehende Beispielversuch entspricht einem erfindungsgemäßen Verfahren das in einer Anlage durchgeführt wird, welche schematisch in Fig. 3 aufgezeigt ist. Es werden wie in dem vorstehenden Verfahren nach dem Stand der Technik 5,3 kg wässrige Ameisensäure hergestellt. Das Reaktionsprodukt des Synthesereaktors 6 enthält neben Methylformiat auch 25 Gew.-% Methanol. Die Aufkonzentrierung des Methylformiats erfolgt in einer als "reine" Abtriebskolonne ausgebildeten Destillationskolonne 8, die lediglich 15 Böden aufweist. Das aus dem Synthesereaktor entnommene Gemisch wird dabei auf den obersten Boden aufgegeben, so daß die Kolonne keinen Verstärkungsteil 11 aufweist. In dem Sumpfverdampfer der Destillationskolonne 8 werden 0,6 kW Wärme - 33 % weniger als bei dem entsprechenden Verfahren nach dem Stand der Technik - eingetragen, am entsprechenden Kondensator werden 0,73 kW -23 % weniger als bei dem Verfahren nach dem Stand der Technik - Wärme abgezogen (damit ist die Einsparung von Kühlwasser verbunden). Mit Hilfe dieser apparativen Anordnung wird Methylformiat auf 89 Gew.-% aufkonzentriert. Der erhaltene Strom wird an der Zulaufstelle 9 in die Destillationseinrichtung 2 eingeführt. Die Destillationseinrichtung 2 unterscheidet sich hinsichtlich der, die beim Vergleichsversuch eingesetzt wird nur durch die Zulaufstelle 9. Diese befindet sich auf dem Boden 65. Der Energieeintrag durch den Sumpfverdampfer der Destillationseinrichtung 7 entspricht dem des Vergleichsversuchs. Die Methylformiatkonzentration an der Entnahmestelle 13 beträgt 95 Gew.-%.

Die als "reine" Abtriebskolonne ausgebildete Destillationskolonne 8 weist nur die halbe Bauhöhe im Vergleich zur der im Vergleichsversuch eingesetzten Destillationskolonne 8 auf. Aufgrund des geringen notwendigen Energieeintrags können die Wärmeüberträger (Verdampfer/Kondensator) um 30 % kleiner dimensioniert werden. Der Kolonnendurchmesser kann um 10 % verringert werden.

Es ist deutlich zu erkennen, daß das erfindungsgemäße Verfahren gegenüber dem Verfahren nach dem Stand der Technik hinsichtlich der Energie- und Investitionskosten deutlich verbessert ist.

## Patentansprüche

1. Verfahren zur Gewinnung von wasserfreier oder weitgehend wasserfreier Ameisensäure, bei dem man
i) Methylformiat der Hydrolyse unterwirft,
ii) vom erhaltenen Hydrolysegemisch Methanol sowie überschüssiges Methylformiat abdestilliert,
iii) das Ameisensäure und Wasser enthaltende Sumpfprodukt der Destillation (ii) in einer Flüssig-flüssig-Extraktion mit einem hauptsächlich die Ameisensäure aufnehmenden Extraktionsmittel extrahiert,
iv) die hierbei erhaltene Ameisensäure, Extraktionsmittel und eine Teilmenge des Wassers aufweisende Extraktphase einer Destillation unterwirft,
v) das bei dieser Destillation erhaltene Wasser und eine Teilmenge der Ameisensäure enthaltende Kopfprodukt in den unteren Teil der Destillationseinrichtung der Stufe (ii) zurückführt,
vi) das vorwiegend Extraktionsmittel und Ameisensäure enthaltende Sumpfprodukt der Destillationsstufe (iv) destillativ in wasserfreie oder weitgehend wasserfreie Ameisensäure und das Extraktionsmittel auftrennt und
vii) das die Stufe (vi) verlassende Extraktionsmittel in den Verfahrensgang zurückführt,
**dadurch gekennzeichnet, daß** vor der Hydrolyse des Methylformiats (Stufe i)), Methanol aufweisendes Methylformiat in die zur Durchführung der Stufe ii) vorgesehene Destillationseinrichtung geführt wird, wobei sich die entsprechende Zufuhrstelle des Methanol enthaltenden Methylformiats in der Destillationseinrichtung oberhalb der Entnahmestelle für Methanol und unterhalb der Entnahmestelle für Methylformiat befindet und an der Entnahmestelle für Methylformiat an Methanol abgereichertes Methylformiat erhalten wird, das nachfolgend der Stufe i) zugeführt wird.

2. Verfahren nach Anspruch 1, **dadurch gekennzeichnet, daß** man das Methanol aufweisende Methylformiat erhält, indem man dem Synthesereaktor entnommenes, Methylformiat und Methanol enthaltendes Gemisch einer bevorzugt als Abtriebskolonne ausgebildeten Destillationskolonne zuführt, dort eine Teilmenge des Methanols abtrennt, und am oberen Ende der Destillationskolonne das Methanol aufweisende Methylformiat entnimmt.

3. Verfahren nach Anspruch 2, **dadurch gekennzeichnet, daß** die Destillationskolonne keinen Verstärkungsteil aufweist.

4. Verfahren nach Anspruch 2 oder 3, **dadurch gekennzeichnet, daß** die Destillationskolonne im oberen Teil keinen Rücklauf aufweist.

5. Verfahren nach einem der Ansprüche 1 bis 4, **dadurch gekennzeichnet, daß** die Destillationsschritte ii) und iv) in einer einzigen Destillationseinrichtung durchgeführt werden.

6. Vorrichtung zur Durchführung des. Verfahrens gemäß einem der Ansprüche 1 bis 5, enthaltend
α) einen Synthesereaktor (6)
β) einen Hydrolysereaktor (1)
χ) eine Destillationseinrichtung (2) zur Durchführung der Stufe ii), aufweisend eine Zufuhrstelle (9) für Methanol enthaltendes Methylformiat und Entnahmestellen (12; 13) für Methanol und Methylformiat,
δ) eine Destillationseinrichtung (4) zur Durchführung der Stufe iv),
ε) eine Extraktionseinrichtung (3) und
φ) eine Destillationseinrichtung (5) zur Durchführung der Stufe vi).

7. Vorrichtung nach Anspruch 6, enthaltend die Elemente α) bis φ) und eine bevorzugt als Abtriebskolonne ausgebildete Destillationskolonne (8).

8. Vorrichung nach Anspruch 6 oder 7, **dadurch gekennzeichnet, daß** die Destillationseinrichtung (2) zur Durchführung der Stufe β) und die Destillationseinrichtung (4) zur Durchführung der Stufe δ) in einer einzigen Destillationseinrichtung (7) angeordnet sind.

## Claims

1. A process for obtaining anhydrous or substantially anhydrous formic acid, in which
i) methyl formate is subjected to hydrolysis,
ii) methanol and excess methyl formate are distilled off from the resultant hydrolysis mixture,
iii) the bottom product from distillation ii), which comprises formic acid and water, is extracted in a liquid-liquid extraction with an extractant which principally takes up the formic acid,
iv) the resultant extract phase, comprising formic acid, extractant and some of the water, is subjected to distillation,
v) the top product obtained in this distillation, which comprises water and some of the formic acid, is fed back into the lower part of the distillation device in step ii),
vi) the bottom product from distillation step iv), which comprises predominantly extractant and formic acid, is separated by distillation into anhydrous or substantially anhydrous formic acid and the extractant, and
vii) the extractant leaving step vi) is fed back into the process, which comprises feeding methanol-containing methyl formate into the distillation device proposed for carrying out step ii) before the hydrolysis of the methyl formate (step i)), where the corresponding feed point for the methanol-containing methyl formate in the distillation device is located above the removal point for methanol and below the removal point for methyl formate, and methyl formate with a reduced methanol content is obtained at the removal point for methyl formate and is subsequently fed to step i).

2. A process as claimed in claim 1, wherein the methanol-containing methyl formate is obtained by feeding the mixture of methyl formate and methanol removed from the synthesis reactor to a distillation column, preferably in the form of a stripping column, separating off some of the methanol therein, and removing the methanol-containing methyl formate at the upper end of the distillation column.

3. A process as claimed in claim 2, wherein the distillation column has no rectifying section.

4. A process as claimed in claim 2 or 3, wherein the distillation column has no return in the upper section.

5. A process as claimed in one of claims 1 to 4, wherein distillation steps ii) and iv) are carried out in a single distillation device.

6. An apparatus for carrying out a process as claimed in one of claims 1 to 5, comprising
α) a synthesis reactor (6),
β) a hydrolysis reactor (1),
χ) a distillation device (2) for carrying out step ii), having a feed point (9) for methanol-containing methyl formate and removal points (12; 13) for methanol and methyl formate,
δ) a distillation device (4) for carrying out step iv),
ε) an extraction device (3), and
φ) a distillation device (5) for carrying out step vi).

7. An apparatus as claimed in claim 6, comprising elements α) to φ) and a distillation column (8), preferably in the form of a stripping column.

8. An apparatus as claimed in claim 6 or 7, wherein the distillation device (2) for carrying out step β) and the distillation device (4) for carrying out step δ) are arranged in a single distillation device (7).

## Revendications

1. Procédé d'obtention d'acide formique anhydre ou essentiellement anhydre, dans lequel :
i) on soumet le formiate de méthyle à hydrolyse,
ii) on distille le méthanol ainsi que de formiate de méthyle en excès du mélange d'hydrolyse obtenu,
iii) on extrait le produit de fond de la distillation (ii), contenant l'acide formique et l'eau, dans une extraction liquide-liquide avec un agent d'extraction fixant principalement l'acide formique,
iv) on soumet l'acide formique ainsi obtenu, l'agent d'extraction et une quantité partielle de la phase extraite contenant de l'eau à une distillation,
v) on renvoie l'eau obtenue de cette distillation et une quantité partielle du produit de tête contenant de l'acide formique dans la partie inférieure de l'installation de distillation de l'étape (ii),
(vi) on sépare le produit de fond contenant essentiellement l'agent d'extraction et l'acide formique, de l'étape de distillation (iv), par distillation en acide formique anhydre ou essentiellement anhydre et en l'agent d'extraction, et
vii) on renvoie l'agent d'extraction quittant l'étape (vi) dans le processus,
**caractérisé en ce qu'**avant l'hydrolyse du formiate de méthyle (étape i)), du formiate de méthyle contenant du méthanol est amené dans le dispositif de distillation prévu pour réaliser l'étape ii), où le site d'alimentation du formiate de méthyle contenant du méthanol se trouve dans l'installation de distillation, au-dessus du site de prélèvement du méthanol et en-dessous du site de prélèvement du formiate de méthyle et on obtient au site de prélèvement du formiate de méthyle, un formiate de méthyle appauvri en méthanol, qui est ramené ensuite à l'étape i).

2. Procédé selon la revendication 1, **caractérisé en ce que** l'on obtient le formiate de méthyle présentant du méthanol **en ce que** l'on amène le mélange contenant le formiate de méthyle et le méthanol, prélevé au réacteur de synthèse, dans une colonne de distillation formée de préférence, sous la forme d'une colonne de rectification, là une quantité partielle du méthanol est séparée et à l'extrémité supérieure de la colonne de distillation, on prélève le formiate de méthanol présentant du méthanol.

3. Procédé selon la revendication 2, **caractérisé en ce que** la colonne de distillation ne présente pas de partie de concentration.

4. Procédé selon la revendication 2 ou 3, **caractérisé en ce que** la colonne de distillation ne présente pas de reflux dans la partie supérieure.

5. Procédé selon l'une quelconque des revendications 1 à 4, **caractérisé en ce que** les étapes de distillation ii) et iv) sont réalisées dans un seul dispositif de distillation. '

6. Dispositif de réalisation du procédé selon l'une quelconque des revendications 1 à 5, contenant :
α) un réacteur de synthèse (6)
β) un réacteur d'hydrolyse (1)
χ) un dispositif de distillation (2) pour réaliser l'étape ii), présentant un site d'alimentation (9) pour le formiate de méthyle contenant le méthanol et un site de prélèvement (12 ; 13) pour le méthanol et le formiate de méthyle,
δ) un dispositif de distillation (4) pour réaliser l'étape iv),
ε) un dispositif d'extraction (3), et
φ) un dispositif de distillation (5) pour réaliser l'étape vi).

7. Dispositif selon la revendication 6, contenant les élément α) à φ) et une colonne de distillation (8) formée de préférence comme une colonne de rectification.

8. Dispositif selon la revendication 6 ou 7, **caractérisé en ce que** le dispositif de distillation (2) pour réaliser l'étape β) et le dispositif de distillation (4) pour réaliser l'étape δ) sont disposés dans un seul dispositif de distillation (7).
